# EUROPÄISCHE PATENTSCHRIFT

(11) **EP 1 558 928 B1**
(45) Veröffentlichungstag und Bekanntmachung des Hinweises auf die Patenterteilung: **25.07.2007**
(21) Anmeldenummer: 03795809.7
(22) Anmeldetag: 06.11.2003
(51) Int. Cl.: G01N 33/50, C12N 5/00

(54) **MEDIUM UND VERFAHREN ZUR MESSUNG DER WIRKSAMKEIT EINER TUMORTHERAPIE**
MEDIUM AND METHOD FOR MEASURING THE EFFICACY OF A TUMOUR THERAPY
MILIEU ET PROCEDE POUR LA MESURE DE L'EFFICACITE D'UNE THERAPIE TUMORALE

(30) Priorität: 07.11.2002 DE 10251879
(43) Veröffentlichungstag der Anmeldung: 03.08.2005
(73) Patentinhaber: Therapyselect GmbH & Co. KG, 69120 Heidelberg (DE)
(72) Erfinder: BABARINA, Alexandra, 81737 München (DE); WALDENMAIER, Dirk, Sebastian, 80469 München (DE); KISCHKEL, Frank, Christian, 32312 Lübbecke (DE)
(74) Vertreter: Dey, Michael
(86) Internationale Anmeldenummer: PCT/EP2003/012428
(87) Internationale Veröffentlichungsnummer: WO 2004/042393

(56) Entgegenhaltungen:
- US-A- 2 002
- US-B1- 6 221 873
- US-B1- 6 452 028
- METZGER R ET AL: "Towards in vitro prediction of an in vivo cytostatic response of human tumor cells with a fast chemosensitivity assay" TOXICOLOGY, LIMERICK, IR, Bd. 166, 14. September 2001 (2001-09-14), Seiten 97-108, XP002228157 ISSN: 0300-483X
- HAFNER FRANK: "Cytosensor(R) Microphysiometer: Technology and recent applications" BIOSENSORS & BIOELECTRONICS, ELSEVIER SCIENCE PUBLISHERS, BARKING, GB, Bd. 15, Nr. 3-4, Juni 2000 (2000-06), Seiten 149-158, XP002183725 ISSN: 0956-5663 in der Anmeldung erwähnt
- MP biomedicals catalogue extract: product [12332] 1 x Dulbecco's modification of eagle's medium with 4500 mg/l dextrose without L-glutamine.
- MORTON: IN VITRO, Bd. 6, Nr. 2, 1970, Seiten 89-108,

## Beschreibung

Die Erfindung betrifft ein Medium zur Messung der Wirksamkeit einer Tumortherapie an Einzelzellsuspensionen sowie ein Verfahren zur Messung der Wirksamkeit einer Tumortherapie unter Verwendung dieses Mediums.

Um die Wirksamkeit von Tumortherapien zu testen, wäre es wünschenswert, an einer isolierten Tumorzelle den Einfluss einer Tumortherapie auf die Lebensfähigkeit dieser Zelle bestimmen zu können. Ein solches Verfahren würde es ermöglichen, bei einem Patienten vorab festzustellen, ob eine bestimmte Therapie bei seinem speziellen Tumor wirksam ist oder nicht. Hierzu wären Tumorteile zu entnehmen, durch enzymatischen Verdau in eine Einzelzellsuspension zu überführen und dann die Lebenstätigkeit der so erhaltenen Einzelzellen unter dem Einfluss der Therapie, typischerweise also des Chemotherapeutikums, zu bestimmen. Ein Vergleich der Stoffwechselaktivität von nicht behandelten Tumorzellen mit der Stoffwechselaktivität von therapeutisch behandelten Tumorzellen lässt dann die Wirksamkeit der Therapie anhand der verringerten Stoffwechselaktivität der behandelten Zelle im Vergleich zur unbehandelten Tumorzelle, direkt bestimmen. Auf diese Weise wäre es möglich innerhalb kürzester Zeit bei einem bestimmten Tumor festzustellen, welche Therapie geeignet oder weniger geeignet ist zur Behandlung des Patienten.

Ein entscheidendes Problem für ein derartiges Verfahren ist es jedoch, in der Zellsuspension die Bedingungen so einzustellen, dass die vereinzelten Tumorzellen eine maximale Stoffwechselaktivität entfalten können. Erst dadurch kann eine Verringerung der Stoffwechselaktivität noch mit ausreichender Genauigkeit gemessen werden. Aus einem kompakten Tumorgewebe durch enzymatischen Verdau hergestellte Einzelzellsuspensionen der Tumorzellen weisen jedoch in den bekannten Medien äußerst geringe Stoffwechselaktivitäten auf, sodass es nur sehr schwer oder gar nicht möglich ist, eine weitere Verringerung der Stoffwechselaktivität mit hinreichender Genauigkeit zu bestimmen.

Der Erfindung liegt die Aufgabe zugrunde, ein Zellkulturmedium zur Verfügung zu stellen, welches auf diese Weise hergestellten Einzelzellsuspensionen von Tumoren eine maximale Stoffwechselaktivität und Lebensdauer zu verleihen und ein Verfahren zur Beurteilung einer Tumortherapie mit diesem Medium zu schaffen.

Gelöst wird diese Aufgabe erfindungsgemäß durch ein Medium zur Messung der Wirksamkeit einer Tumortherapie an Einzelzellsuspensionen enthaltend die essenziellen Aminosäuren, Vitamine, Salze und Kohlenstoffdonatoren, dadurch gekennzeichnet, dass das Medium 0,1 bis 1 mM Puffer pH 7,0 bis 7,4, 2 bis 10 g/l Glucose und 2 bis 5 mM Glutamin als Kohlenstoffquellen und 5 bis 20 % fötales Kälberserum enthält, wobei das Medium Keine anderen Kohlenstoffquellen enthält.

US 6,221,873 beschreibt die Verwendung eines Mediums basierend auf DMEM (Dulbecco's modified Eagle's Medium), welches mehr als 44. mM an Puffer sowie Pyruvat enthält. Metzger et al. (Toxicology 166 (2001), 97-108) beschreibt die Verwendug eines mediums basierend auf RPMI (ImM Phosphatpuffer), welches Kein Fötales Kälberserum enthält.

Von bekannten Medien unterscheidet sich das erfindungsgemäße Medium durch eine sehr geringe Pufferkapazität, einen höheren Gehalt an Glucose, Glutamin und fötalem Kälberserum. Andere Kohlenstoffquellen außer Glucose und Glutamin und weitere Puffer enthält das Medium nicht.

Die Zusammensetzung des erfindungsgemäßen Mediums richtet sich in gewissem Maße nach den metabolischen Eigenschaften der in dem untersuchten Tumor enthaltenen Zellen. Die Einstellung der Variablen im erfindungsgemäßen Medium strebt dabei an für die Tumorzellen die Bedingungen der Glycolyse einzustellen, da hierbei eine hohe pH-Wert-Änderung zu erwarten ist. Gleichzeitig sollen die in der Suspension unvermeidlicherweise ebenfalls enthaltenen normalen Zellen nach Möglichkeit im aeroben Metabolismus gehalten werden und dadurch eine vernachlässigbar geringe Wirkung auf den pH-Wert ausüben.

Da Tumorzellen mehr Glucose verbrauchen als viele nicht-Tumorzellen und andererseits bei Tumorzellen die Erreichung einer möglichst hohen Glycolyse angestrebt wird, bei der die Protonenbildung und damit die Ansäuerung des Mediums pro produziertes ATP-Mofekül am höchsten ist, beträgt die Glucosemenge im erfindungsgemäßen Medium mehr als 2 g/l, besonders bevorzugt 4 bis 6 g/l Glucose. Andere Kohlenstoffquellen, ausgenommen das als essenzieller Bestandteil des Mediums vorliegende Glutamin, sollen nicht enthalten sein. Typische Beispiele für derartige unerwünschte Kohlenstoffquellen sind Pyruvat, Lipide und ähnliche bekannte Kohlenstoffquellen in Nährmedien. Insbesondere Lipide erwiesen sich in Kombination mit Glutamin als schädlich, da hierdurch die Anfangsaktivität aller Tumorzellen stark gesenkt wird.

Die besten Ergebnisse hinsichtlich Steigerung der Aktivität der Tumorzellen bei gleichzeitig geringstmöglichem Einfluss der nicht-Tumorzellen auf die pH-Wertänderung ergab daher einen Gehalt von 5 g/l Glucose, 2 mM Glutamin und 10 Vol.-% fötales Kälberserum, wobei jeder dieser drei Werte ± 10 % schwanken kann.

Das erfindungsgemäße Medium kann durch Aufbau aus den Komponenten hergestellt werden. Beispielsweise können übliche Vitaminmischungen und Proteinhydrolysate als Quelle der essenziellen Aminosäuren mit den oben genannten essenziellen Mengen an Glucose, Glutamin, Puffer und fötalem Kälberserum aufgebaut werden. Alternativ kann auch von bekannten Medien ausgegangen und deren Zusammensetzung durch Zugabe der wichtigen Mengen an essenziellen Bestandteilen wie oben erläutert, ergänzt werden. Insbesondere eignet sich hierzu das RUN-Medium als Ausgangsbasis. Ferner kann das Medium Antibiotika enthalten.

Ein weiterer Gegenstand der Erfindung ist ein Verfahren zur Messung der Wirksamkeit einer Tumortherapie an Einzelzellsuspensionen von Tumorzellen durch Vergleich der Säurebildung in einem Medium bei therapierten und nicht therapierten Tumorzellen, welches dadurch gekennzeichnet ist, dass man die Messung in einem Medium wie vorstehend beschrieben durchführt. Das Verfahren eignet sich gut zur Durchführung unter Verwendung handelsüblicher Vorrichtungen wie beispielsweise dem Cytosensor^{®}-Mikrophysiometer der Firma Molecular Devices Corp., Sunnyvale, CA., USA, ist aber nicht hierauf beschränkt.

Letztere Vorrichtung und deren Verwendung zur Analyse von Zellmembrangebundenen Rezeptoren ist aus Biosensors & Bioelectronics 15 (2000) 149-158 bekannt, wobei die durch die physiologischen Effekte solcher Rezeptoren hervorgerufenen pH-Änderungen gemessen werden als Maß für die metabolischen Effekte.

Das erfindungsgemäße Verfahren zur Messung der Wirksamkeit einer Tumortherapie wird nachfolgend beispielsweise unter Verwendung des Cytosensor^{®}-Mikrophysiometers beschrieben.

Zur Durchführung des erfindungsgemäßen Verfahrens unter Verwendung des erfindungsgemäßen Mediums wird eine Probe des zu untersuchenden Tumors entnommen und in bekannter Weise durch enzymatischen Verdau in eine Einzelzellensuspension überführt. Die Zellen werden aus dem Verdaumedium abgetrennt und dann deren pH-Kurve in dem erfindungsgemäßen Medium aufgenommen. Ein typisches Medium gemäß der Erfindung weist z.B. die aus der nachstehenden Tabelle 1 zu entnehmende Zusammensetzung auf.

**Tabelle 1**

| Konzentration | Substanz |
|---|---|
| mg/l | |
| 4,45 | Alanin, L- |
| 147,5 | Arginin L-, HCL |
| 7,5 | Asparagin, L-, H2O |
| 6,65 | Asparaginsäure, L- |
| 24 | Cystin, L- |
| 17,56 | Cystein, L-, HCL, H2O |
| 7,35 | Glutaminsäure, L- |
| 18,75 | Glycin |
| 31,48 | Histidin, L-, HCl H2O |
| 20 | Hydroxyprolin, L-4- |
| 54,45 | Isoleucin, L- |
| 59,05 | Leucin, L- |
| 91,25 | Lysin, L-HCl |
| 17,24 | Methionin, L- |
| 35,48 | Phenylalanin, L- |
| 17,25 | Prolin, L- |
| 26,25 | Serin, L- |
| 53,45 | Threonin, L- |
| 9,02 | Tryptophan, L- |
| 38,7 | Tyrosin, L- |
| 52,85 | Valin, L- |
| 2000 | D + Glucose |
| 12,6 | Inosit, myo-, inosit, meso- |
| 0,00365 | Biotin, D- |
| 2,24 | Calcium D-Pantothenat |
| 8,98 | Cholinchlorid |
| 1 | Glutathion, L-, reduziert |
| 2,02 | Nicotinsäureamid |
| 2,031 | Pyridoxin, HCL |
| 2,17 | Thiaminchlorid, Thiamin HCL |
| 0,01 | Tocopherolsuccinat DL-alfa |
| 0,1 | Vitamin A-acetat |
| 0,68 | Vitamin B 12 (Cyanocobalamin) |
| 2 | Vitamin C (Ascorbinsäure) |
| 0,1 | Vitamin D2 |
| 8,1 | Phenolrot |
| 154 | Calciumchlorid 2 H2O |
| 0,05 | Eisen (III) nitrat, 9 H2O |
| 0,417 | Eisen (II) Sulfat, 7 H2O zur Analyse |
| 311,8 | Kaliumchlorid |
| 0,00125 | Kupfer (II) sulfat, 5 H2O |
| 100 | Magnesiumsulfat, 7 H2O |
| 61 | Magnesiumchlorid, 6 H2O |
| 6999,5 | Natriumchlorid |
| 14,2 | di-Natriumhydrogenphosphat (anhyd.) |
| 0,43 | Zinksulfat, 7 H2O |
| 2 mM | Glutamin |
| 10 Vol.-% | fötales Kälberserum |
| 100 Units/ml | Penicillin |
| 100 µg/ml | Streptomycin |
| 100 µg/ml | Kanamycin |
| 2,5 µg/ml | Fungizone |

Hierzu werden die so erhaltenen Einzelzellen dann vorzugsweise auf einer pH-Elektrode immobilisiert. Die Immobilisierung kann durch geeignete Substanzen wie sie in der Zellkultur üblich sind, erfolgen. Als gut geeignet hat sich Agarose herausgestellt, welches die gewonnen Einzelzellen auf der Elektrodenoberfläche immobilisieren kann aber auch andere in der Zellkultur verwendete Immobilisierungsmittel sind brauchbar.

Das verwendete Gerät weist acht Kanäle auf, in denen simultan der pH-Wert in Durchflusszellen bestimmt werden kann. In jedem Kanal ist eine pH-Elektrode angeordnet, auf der sich die immobilisierte Zellsuspension befindet. Über diese immobilisierte Zellsuspension wird dann durch eine Pumpe für einen geeigneten Zeitraum das erfindungsgemäße Medium gepumpt und gleichzeitig mit der pH-Messung begonnen. In einem typischen Fall dauert ein Messzyklus 120 Sekunden. Davon wird 90 Sekunden lang nur Medium zugepumpt und jede Sekunde ein pH-Wert bestimmt. Danach wird über 30 Sekunden die pH-Wert-Änderung gemessen. Dieser Vorgang wird über einen längeren Zeitraum, in der Regel 14 bis 24 Stunden, fortgesetzt, sodass sich schließlich eine pH-Wert-Kurve ergibt, welche der metabolischen Aktivität der immobilisierten Zellen direkt äquivalent ist.

Im oben angegebenen Gerät finden sich wie oben erwähnt, acht parallele Kanäle. Zur Bestimmung der Wirksamkeit einer Tumortherapie wird zweckmäßig in einem Kanal zur Bestimmung einer Basismesskurve nur erfindungsgemäßes Medium durchgepumpt, in den anderen Kanälen können verschiedene Antitumormittel (Cytostatika) gleichzeitig bestimmt werden. Da die Cytostatika, wenn sie gegenüber den hier verwendeten Tumorzellen eine Wirksamkeit entfalten, zu einer Herabsetzung der metabolischen Aktivität führen, erhält man eine pH-Wert-Kurve über die Zeit, welche in Folge der verringerten metabolischen Aktivität durch die Cytostatikaeinwirkung eine geringere pH-Wert-Änderung zur Folge hat. Aus dem Vergleich der Kurvenneigungen, die dann bei den in Gegenwart von Cytostatika gemessenen Kurven im Vergleich zu der Cytostatika-freien Messkurve gewonnen werden, lässt sich direkt die Aktivität der jeweils untersuchten cytostatisch wirksamen Verbindungen bestimmen. In gleicher Weise ist es auch möglich, nicht in den verschiedenen Kanälen verschiedene cytostatisch oder cytotoxisch wirkamen Verbindungen zu untersuchen sondern für ein bestimmtes Cytostatikum unterschiedliche Konzentrationen hinsichtlich ihrer Wirksamkeit zu untersuchen. Letztere Ausführungsform hat den Vorteil, dass evtl. Einflüsse des untersuchten Cytostatikums auf den pH-Wert durch Anpassung des Mediums im Rahmen der erfindungsgemäßen Grenzwerte insbesondere hinsichtlich der Pufferkapazität ausgeglichen werden können. Die Pufferkapazität als solche wird möglichst gering gehalten, darf jedoch 0,1 mM nicht unterschreiten. Vorzugsweise wird die Puffermenge möglichst nahe an diesem unteren Grenzwert eingestellt. Bei Cytostatika, die selbst den pH-Wert beeinflussen können, kann jedoch dann die Puffermenge erhöht werden bis maximal 1 mM.

Erfindungsgemäß wird es möglich, innerhalb sehr kurzer Zeit die Wirksamkeit einer Tumortherapie bei einem Patienten extrakorporal zu messen und aufgrund der Messergebnisse dann die Behandlung entsprechend einzustellen. Damit wird nicht nur ein entscheidender Zeitgewinn bei der Behandlung des Patienten ermöglicht sondern auch das Risiko unerwünschter Nebenwirkungen der Therapie ohne entsprechende Wirksamkeit gegenüber dem Tumor selbst verringert oder ganz vermieden. Dies bedingt einen erheblichen Fortschritt bei der Tumortherapie.

## Patentansprüche

1. Medium zur Messung der Wirksamkeit einer Tumortherapie an Einzelzellsuspensionen, enthaltend die essenziellen Aminosäuren, Vitamine, Salze und Kohlenstoffdonatoren,
**dadurch gekennzeichnet,**
**dass** das Medium 0,1 bis 1 mM Puffer pH 7,0 bis 7,4,
2 bis 10 g/l Glucose und 2 bis 5 mM Glutamin als Kohlenstoffquellen und
5 bis 20 Vol.-% fötales Kälberserum enthält, wobei
das Medium Keine anderen Kohlenstoffquellen enthält.

2. Medium nach Anspruch 1,
**dadurch gekennzeichnet,**
**dass** es als Puffer Phosphatpuffer enthält.

3. Medium mach Anspruch 1 oder 2,
**dadurch gekennzeichnet,**
**dass** es 8 bis 12 Vol.-% fötales Kälberserum enthält.

4. Medium nach einem der Ansprüche 1 bis 3,
**dadurch gekennzeichnet,**
**dass** das Medium 5 g/l Glucose, 2 mM Glutamin und 10 Vol.-% fötales Kälberserum enthält, wobei jeder dieser Werte um 10 % abweichen kann.

5. Verfahren zur Messung der Wirksamkeit einer Tumortherapie an Einzelzellsuspensionen von Tumorzellen durch Bestimmung der Säurebildung in einem Medium in Gegenwart und in Abwesenheit einer cytostatisch oder cytotoxisch wirkamen Substanz,
**dadurch gekennzeichnet,**
**dass** man die Messung in einem Medium nach einem der Ansprüche 1 bis 4 durchführt.

6. Verfahren nach Anspruch 5,
**dadurch gekennzeichnet,**
**dass** man die Messung mittels einer pH-Elektrode, auf welcher die Einzelzellsuspension immobilisiert ist, durchführt unter Verwendung einer Durchflusszelle, welche von dem erfindungsgemäßen Medium durchströmt wird.

7. Verfahren nach Anspruch 5 oder 6,
**dadurch gekennzeichnet,**
**dass** man das Medium durch die Durchflusszelle pumpt bis sich ein konstanter pH-Wert eingestellt hat und dann durch Messung in kurzen Abständen die Änderung des pH-Wertes bei stehendem Medium misst, das Medium danach aus der Messzelle entfernt und mit dem Messzyklus solange wieder von vorne beginnt, bis man die pH-Wert-Änderung über einen längeren Zeitraum bestimmt hat.

8. Verfahren nach Anspruch 7,
**dadurch gekennzeichnet,**
**dass** man das Medium 1 1/2 bis 2 1/2 Minuten lang zuführt und misst und dann mit frischem Medium den Vorgang 14 bis 24 Stunden lang wiederholt.

9. Verfahren nach Anspruch 8,
**dadurch gekennzeichnet,**
**dass** man das Verfahren in einem Mehrkanalgerät durchführt, wobei ein Kanal von Medium ohne Cytostatikum beschickt wird und die anderen Kanäle mit dem gleichen Medium, welches das zu untersuchende Cytostatikum enthält, beschickt werden.

## Claims

1. Medium for measuring the efficacy of a tumour therapy on single cell suspensions, containing the essential amino acids, vitamins, salts and carbon donors,
**characterized in that**
the medium contains 0.1 to 1 mM buffer of pH 7.0 to 7.4,
2 to 10 g/l glucose and 2 to 5 mM glutamine as carbon sources and
5 to 20 % by volume foetal calf serum, wherein the medium contains no other carbon sources.

2. Medium according to claim 1,
**characterized in that**
it contains phosphate buffer as the buffer.

3. Medium according to claim 1 or 2,
**characterized in that**
it contains 8 to 12 % by volume foetal calf serum.

4. Medium according to one of the claims 1 to 3,
**characterized in that**
the medium contains 5 g/l glucose, 2 mM glutamine and 10 % by volume foetal calf serum, where each of these values can vary by 10 %.

5. Method for measuring the efficacy of a tumour therapy on single cell suspensions of tumour cells by determining the acid formation in a medium in the presence and in the absence of a substance having cytostatic or cytotoxic activity,
**characterized in that**
the measurement is carried out in a medium according to one of the claims 1 to 4.

6. Method according to claim 5,
**characterized in that**
the measurement is carried out by means of a pH electrode on which the single cell suspension is immobilized using a flow cell through which the medium according to the invention flows.

7. Method according to claim 5 or 6,
**characterized in that**
the medium is pumped through the flow cell until a constant pH has been set and the change in the pH with the medium stationary is then measured by measuring at short intervals, the medium is subsequently removed from the measuring cell and the measurement cycle is started from the beginning again until the pH change has been determined over a long period.

8. Method according to claim 7,
**characterized in that**
the medium is fed in for 1 ½ to 2 ½ minutes and a measurement is carried out and then the procedure is repeated with fresh medium for 14 to 24 hours.

9. Method according to claim 8,
**characterized in that**
the method is carried out in a multichannel instrument, wherein one channel is filled with a medium without a cytostatic agent and the other channel is filled with the same medium which contains the cytostatic agent to be examined.

## Revendications

1. Milieu pour mesurer l'efficacité d'une thérapie tumorale sur des suspensions de cellules individuelles, contenant les aminoacides essentiels, des vitamines, des sels et des donneurs de carbone, **caractérisé en ce que** le milieu contient 0,1 à 1 mM de tampon pH 7,0 à 7,4,
2 à 10 g/l de glucose et 2 à 5 mM de glutamine comme sources de carbone
et
5 à 20 vol.% de sérum foetal bovin, où le milieu ne contient aucune autre source de carbone.

2. Milieu selon la revendication 1 **caractérisé en ce qu'**il contient comme tampon du tampon phosphate.

3. Milieu selon la revendication 1 ou 2 **caractérisé en ce qu'**il contient 8 à 12 vol. % de sérum foetal bovin.

4. Milieu selon l'une des revendications 1 à 3 **caractérisé en ce que** le milieu contient 5 g/l de glucose, 2 mM de glutamine et 10 vol. % de sérum foetal bovin, où chacune de ces valeurs peut s'écarter de 10 %.

5. Procédé pour mesurer l'efficacité d'une thérapie tumorale sur des suspensions de cellules individuelles de cellules tumorales par détermination de la formation d'acide dans un milieu en présence et en l'absence d'une substance efficace du point de vue cytostatique ou cytotoxique, **caractérisé en ce que** l'on réalise la mesure dans un milieu selon l'une des revendications 1 à 4.

6. Procédé selon la revendication 5 **caractérisé en ce que** l'on réalise la mesure au moyen d'une électrode de pH, sur laquelle est immobilisée la suspension de cellules individuelles, en utilisant une cuve à circulation qui est traversée par le milieu selon l'invention.

7. Procédé selon la revendication 5 ou 6 **caractérisé en ce que** l'on pompe le milieu dans la cuve à circulation jusqu'à ce qu'un pH constant se soit établi puis on mesure la variation du pH tandis que le milieu est immobile en mesurant à de courts intervalles, ensuite on retire le milieu de la cuve de mesure et on recommence le cycle de mesure depuis le début jusqu'à ce que l'on ait déterminé la variation du pH sur une durée plus longue.

8. Procédé selon la revendication 7 **caractérisé en ce que** l'on introduit le milieu et mesure pendant 1 ½ à 2 ½ minutes puis on répète le processus avec du milieu frais pendant 14 à 24 heures.

9. Procédé selon la revendication 8 **caractérisé en ce que** l'on met en oeuvre le procédé dans un appareil à canaux multiples où un canal est alimenté avec du milieu sans cytostatique et les autres canaux sont alimentés avec le même milieu qui contient le cytostatique à étudier.
